# EUROPEAN PATENT SPECIFICATION

(11) **EP 2 738 249 B1**
(45) Date of publication and mention of the grant of the patent: **26.04.2017**
(21) Application number: 12865084.3
(22) Date of filing: 07.03.2012
(51) Int. Cl.: C12N 5/07, C12N 11/04, A61L 27/52, A61L 27/54, C12N 5/071, A61L 27/18, A61L 27/24, A61L 27/38

(54) **METHOD FOR MANUFACTURING IN VITRO VASCULARIZED TISSUE**
VERFAHREN ZUR HERSTELLUNG VON IN VITRO VASKULARISIERTEM GEWEBE
PROCÉDÉ DE FABRICATION D'UN TISSU VASCULARISÉ IN VITRO& xA;

(30) Priority: 11.01.2012 KR 20120003567
(43) Date of publication of application: 04.06.2014
(73) Proprietor: Kang, Gook-Jin, Seoul 138-773 (KR)
(72) Inventor: Kang, Gook-Jin, Seoul 138-773 (KR)
(74) Representative: Rentsch Partner AG
(86) International application number: PCT/KR2012/001668
(87) International publication number: WO 2013/105696

(56) References cited:
- KR-B1- 100 921 487
- NASSER SADR ET AL: "SAM-based cell transfer to photopatterned hydrogels for microengineering vascular-like structures", BIOMATERIALS, ELSEVIER SCIENCE PUBLISHERS BV., BARKING, GB, vol. 32, no. 30, 14 June 2011 (2011-06-14) , pages 7479-7490, XP028261574, ISSN: 0142-9612, DOI: 10.1016/J.BIOMATERIALS.2011.06.034 [retrieved on 2011-06-29]
- NICHOL J W ET AL: "Cell-laden microengineered gelatin methacrylate hydrogels", BIOMATERIALS, ELSEVIER SCIENCE PUBLISHERS BV., BARKING, GB, vol. 31, no. 21, 1 July 2010 (2010-07-01), pages 5536-5544, XP002725666, ISSN: 0142-9612, DOI: 10.1016/J.BIOMATERIALS.2010.03.064 [retrieved on 2010-04-24]
- TSANG ET AL.: 'Three-dimensional tissue fabrication' ADVANCED DRUG DELIVERY REVIEWS vol. 56, 19 July 2004, pages 1635 - 1647, XP004550361
- MOON ET AL.: 'Biomimetic hydrogels with pro-angiogenic properties' BIOMATERIALS vol. 31, 24 February 2010, pages 3840 - 3847, XP026947557
- SAIK ET AL.: 'Biomimetic hydrogels with immobilized EphrinAl for therapeutic angiogenesis' BIOMACROMOLECULES vol. 12, 06 June 2011, pages 2715 - 2722, XP055081312
- Meenakshi Upreti ET AL: "Tumor-Endothelial Cell Three-dimensional Spheroids: New Aspects to Enhance Radiation and Drug Therapeutics", Translational Oncology, vol. 4, no. 6, 1 December 2011 (2011-12-01), pages 365-IN3, XP055244674, United States ISSN: 1936-5233, DOI: 10.1593/tlo.11187
- YONGJI WANG ET AL: "Tissue-specific distributions of alternatively spliced human PECAM-1 isoforms", AMERICAN JOURNAL OF PHYSIOLOGY: HEART AND CIRCULATORY PHYSIOLOGY, vol. 284, no. 3, 1 March 2003 (2003-03-01) , pages H1008-H1017, US ISSN: 0363-6135, DOI: 10.1152/ajpheart.00600.2002

## Description

### [Technical Field]

The present invention relates to a method for manufacturing *in vitro* vascularized tissues, and more particularly, to a method for manufacturing an *in vitro* vascularized tissue that may derive angiogenesis *in vitro* from a corresponding tissue using vascular cells, thus obtaining the *in vitro* vascularized tissue, so as to use the same in *in vitro* research of diseases in regard to vascularized tissues and development of a treatment method thereof.

### [Background Art]

In recent years, as cancer incidence has increased due to aging populations, increases in smoking, changes in dietary patterns, lack of exercise, or the like, development of anticancer medicines for treating cancer has also actively progressed. Anticancer medicines developed in the art have advantageous effects but mostly involve some problems such as toxicity caused by destruction of normal cells, drug tolerance to anticancer medicines, cancer recurrence, or the like, therefore, an improved anticancer medicine based on a novel mechanism is still required. Accordingly, there have been intensive efforts to discover the causes of the occurrence of cancers and progress thereof thus propose that more fundamental solutions to overcome these diseases are required and, in 1971, J. Folkman suggested an anti-angiogenic agent, as a novel alternative idea, in order to prevent growth and metastasis of tumors (see Folkman et al., N. Engl. J. Med., 285, p1182, 1971).

Angiogenesis refers to a process of generating new blood vessels from existing blood vessels, and becomes a cause of various diseases including, for example, metastasis of tumors, diabetic retinopathy, psoriasis, chronic inflammation, ulcers, or the like [Carmeliet et al., Nature, 407, p249, 2000]. In particular, in a case of serious angiogenesis such as that associated with cancer, growth and metastasis of tumors can be efficiently inhibited by simply suppressing such angiogenesis.

Targets for developing an anticancer medicine by suppressing angiogenesis may include, for example, a vascular endothelial growth factor (VEGE), vascular endothelial growth factor receptor (VEGER), basic fibroblast growth factor (bFGF), transfer growth factor alpha and beta (TGF-α, -β), epidermal growth factor (EGF), platelet-derived growth factor (PDGF), or the like.

When administering the foregoing anticancer medicine to a patient, effective anticancer agents vary in term of kinds and dosages depending upon patients thereof, therefore, it is not appropriate to connect the same to data known in academic art or experience of physicians. Further, since such anticancer medicine typically destroys normal cells as well as cancer cells, the number of times anticancer medicine is administered to a patient is limited to 4 times at most, thereby making it impossible to test a variety of kinds or dosages of anticancer medicine.

Accordingly, although anticancer medicine must be directly applied to particular cancer cells in the patient for examination, in order to discover any suitable anticancer medicine, a solution to overcome the above problem has still not been proposed.

Therefore, selection of such anticancer medicines which have such a low success rate is nothing but a probability game involving the life of cancer patients whose days are numbered. However, cancer patients typically have no grounds with which to make a selection except for relying on an uncertain treatment method and adhering to the selection of a physician. Further, despite that the expenses to the patient or his/her family are considerable, the results of the treatment are far from certain.

Further, with regard to selection of appropriate methods for treatment of diseases in vascularized tissues such as the liver, brain, or the like, there is no choice but to rely on the experience of physicians or data known in academic art among a number of treatment methods such as cancer treatment.

In addition, recent radiation methods for cancer treatment widely used in the art may also entail the foregoing problems. Radiation-based cancer treatment may irradiate both of normal cells and cancer cells with radioactive rays whereby the normal cells then rapidly recover, as compared to the cancer cells, wherein the radioactive rays for treatment may include, for example, X-rays, gamma-rays, electron rays, neutron rays, proton rays, baryon rays, or the like, in a range of 1,000,000 volts or more, and a type or dosage of radiation must be different according to patients or body parts of the patient irradiated by the radioactive rays.

That is, although a position of a cancer cell can be identified through CT or MRI, a correct dosage of radiation is actually determined only by the experience of a skilled person. Accordingly, there are still problems in that cancer cells are not destroyed due to an insufficient dosage of radiation, or normal cells are destroyed due to an excessive dosage of radiation, hence potentially causing a patient fatal damage.

The document "Tumor-Endothelial Cell Three-dimensional Spheroids: New Aspects to Enhance Radiation and Drug Therapeutics" [Meetnakshi Upreti ET AL, Translational Oncology, vol. 4, no. 6, 2011-12-01, pages 365-IN3, XP055244674, United States] discloses a system to grow three-dimensional cultures of GFP-4T1 mouse mammary tumor and 2H11 murine endothelial cells in hanging drops of medium *in vitro*. The presence of 2H11 endothelial cells in the three-dimensional cocultures is found to sensitize 4T1-GFP tumor cells to chemotherapy (Taxol) and, at the same time, protect cells from ionizing radiation.

### [Disclosure]

### [Technical Problem]

Accordingly, in order to solve the above-mentioned problems, an object of the present invention is to provide a method for manufacturing *in vitro* vascularized tissues, that uses vascular cells *in vitro* to derive angiogenesis from corresponding tissues, thus obtaining *in vitro* vascularized tissues, so as to use the same in *in vitro* research of diseases in regard to vascularized tissues and development of a method for treatment thereof.

### [Technical Solution]

In order to accomplish the foregoing object, the present invention provides a method for manufacturing *in vitro* vascularized tissues, which includes: supplying a hydrogel mixed with vascularized tissue cells in a container for preparation of tissues; submerging a collection tip having vascular cells adhered thereto in the hydrogel; curing the hydrogel; and supplying a cell culture solution to an upper side of the hydrogel.

The vascular cells are adhered to the collection tip in a cell collection member by submerging the collection tip in a cell culture solution containing the vascular cells. Therefore, it is possible to prepare the vascular cells in large quantities beforehand, thereby reducing a time required for manufacturing *in vitro* vascularized tissues.

In this regard, the container for vascular cells preferably has an inclined part to gather the vascular cells around the collection tip and, more preferably, a collection hole is formed in the center of the inclined part in order to insert the collection tip therein. The collection tip may made a porous material or have a collagen coating applied to an outer surface thereof.

Further, the vascularized tissue cells may be at least one selected from cancer cells, brain cells or liver cells.

Further, the hydrogel may have a cell binding domain while being biodegradable. Therefore, an environment similar to the interior of a human body may be provided in the vascularized tissues. Additionally, the hydrogel may be a natural hydrogel or synthetic hydrogel.

A ratio by weight of the vascular cells to the vascularized tissue cells may range from 1 to 10. That is, increasing an amount of vascularized tissue cells higher than that of the vascular cells may make it easy to identify generation of new blood vessels by a vascular endothelial growth factor (VEGF) provided from the vascularized tissue cells.

### [Advantageous Effects]

According to the method for manufacturing *in vitro* vascularized tissues of the present invention, angiogenesis of cancer cells, liver cells, nerve cells, or the like, using vascular cells *in vitro* may enable *in vitro* mass production of cancer tissues, liver tissues, brain tissues, or the like, simultaneously.

Accordingly, using the *in vitro* vascularized tissues obtained by the present invention, diseases associated with vascularized tissues may be subjected to *in vitro* research and a treatment method of the diseases may be developed. Further, an appropriate method for treating individual patients can be quickly determined.

Therefore, according to the present invention, an effective treatment rate of a disease associated with vascularized tissues such as cancer, liver disease, stroke, dementia, or the like, may increase due to existing anticancer medicines or treatment methods.

Specifically, for radiation-based tumor or cancer treatment, the present invention may also be applied to *in vitro* mass production of cancer tissues, thereby determining a type and dosage of radiation suitable for removal of corresponding cancer cells. As a result, even in radiation-based cancer treatment, side effects caused by incorrect use of radiation may be reduced to thus remarkably improve a cancer treatment rate.

### [Description of Drawings]

FIG. 1 is a flow diagram illustrating a process of adhering vascular cells to a collection tip in a method for manufacturing *in vitro* vascularized tissues according to the present invention.
FIG. 2 is a flow diagram illustrating a process of curing and culturing the prepared vascular cells, as shown in FIG. 1, together with vascularized tissue cells in a hydrogel in the method for manufacturing *in vitro* vascularized tissues according to the present invention.
FIG. 3 is a view illustrating a drug test using the *in vitro* vascularized tissues manufactured by the method for manufacturing *in vitro* vascularized tissues according to the present invention.
FIG. 4 is a perspective view of a cell collection member.

### [Best Mode]

Hereinafter, preferable embodiments of the present invention will be described with reference to the accompanying drawings. Referring to the drawings, like reference characters designate like or corresponding parts throughout the several views. In the embodiments of the present invention, a detailed description of publicly known functions and configurations that are judged to be able to make the purpose of the present invention unnecessarily obscure are omitted.

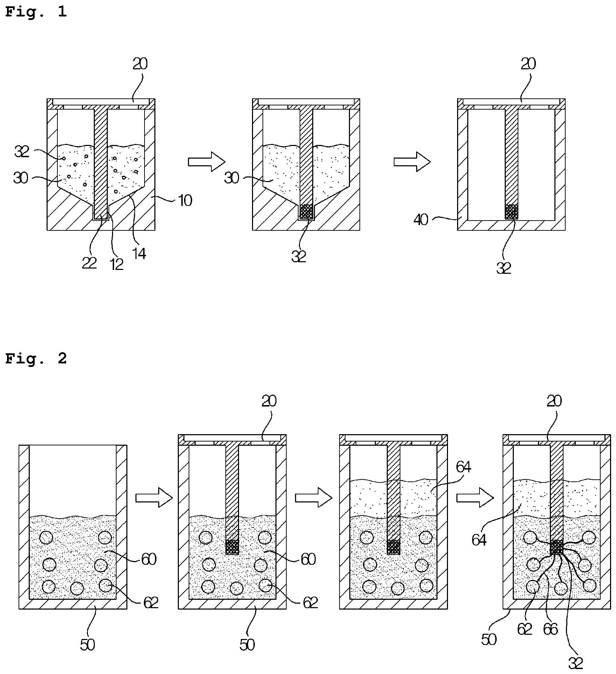

FIG. 1 illustrates a process of adhering vascular cells 32 to a collection tip 22 of a cell collection member 20 in order to prepare vascular cells 32. The vascular cells 32 generally sink in a cell culture solution 30 unless an external force is applied thereto. In the present invention, the vascular cells 32 are collected using the above characteristic.

The vascular cells 32 mixed with the cell culture solution 30 are supplied in the container 10 for vascular cells. That is, the cell culture solution 30 may be injected to the vascular cells 32 or, otherwise, the vascular cells 32 may be supplied to the cell culture solution 30 and mixed with the same. Further, the cell collection member 20 is mounted on the container 10 for vascular cells. The cell culture solution may be any conventional cell culture fluid and, for example, Dulbecco's Modified Eagle's Medium (DMEM) or α-Minimum Essential Medium (α-MEM) may be used.

On the other hand, after mounting the cell collection member 20 on the container 10 for vascular cells, the cell culture solution 30 mixed with vascular cells 32 may be injected thereto.

The container 10 for vascular cells may have a shape that makes it easy to gather the vascular cells 32 around the collection tip 22 of the cell collection member 20. That is, the container 10 for vascular cells may have an inclined part 14 at the bottom thereof wherein the center of the inclined part is lowered further than the bottom of the container. Accordingly, the vascular cells may be gathered in the center of the inclined part 14 through a sloping side of the inclined part 14.

The center of the inclined part 14 is provided with a collection groove 12 so that the vascular cells easily contact the collection tip 22. The collection tip 22 may be inserted into the collection groove 12, and a gap may be formed around the collection groove 12, thus leading the vascular cells 32 to flow therein. Accordingly, it proceeds from a state shown in the left part of FIG. 1 to another state shown in the middle part of FIG. 1. In particular, in order to improve adhesion of the vascular cells 32 to the collection tip 22, the collection tip 22 may be made of a porous material or coated with collagen.

The prepared cell collection member 20 may be directly provided in the container 10 for vascular cells or, otherwise, placed in an alternative container 40 for mounting without a cell culture solution.

Next, as shown in FIG. 2, using the cell collection member 20 having the vascular cells 32 adhered thereto, *in vitro* vascularized tissues may be manufactured.

First, a hydro]gel 60 and vascularized tissue cells 62 are supplied in a container 50 for preparation of tissues. The hydrogel 60 and vascularized tissue cells 62 have been already mixed well, as shown in the left part of FIG. 2. The container 50 for preparation of tissues may be made of a transparent material such as glass or plastic, thereby enabling observation inside the container 50. However, a shape of the container is not particularly limited.

Further, the collection tip 22 is submerged in the hydrogel 60. The collection tip 22 may be submerged in the hydrogel 60 in a coupled state with the cell collection member 20, as shown in FIG. 2. Alternatively, the collection tip 22 may be separated from the cell collection member 20 and submerged alone in the hydrogel 60. In this regard, a ratio by weight of the vascular cells 18 to the vascularized tissue cells 62 may range from 1 to 10. That is, increasing an amount of vascularized tissue cells higher than that of the vascular cells may make it easy to identify generation of new blood vessels by VEGF provided from the vascularized tissue cells.

Accordingly, the vascular cells 32 in the collection tip 22 may be positioned close to the vascularized tissue cells 62 in the hydrogel 60.

The vascularized tissue cells 62 may be selected from cancer cells, brain cells and liver cells, and the vascularized tissue cells 62 may be taken from a patient. For instance, if cancer cells are used, approximately 20,000 to 40,000 cells may be provided.

The vascularized tissue cells may be cultured in an alternative cell culture solution and, after removing the cell culture solution, the cultured tissue cells may be supplied in the container 50 for preparation of tissues. The cell culture solution may be any conventional cell culture fluid and, for example, Dulbecco's Modified Eagle's Medium (DMEM) or α-Minimum Essential Medium (α-MEM) may be used.

The hydrogel 60 must have a cell binding domain while being biodegradable. The foregoing hydrogel 60 may be a natural hydrogel or synthetic hydrogel. The natural hydrogel may include collagen or the like, while the synthetic hydrogel may include, for example, commercial QGel (QGel^{™}, QGel SA, Switzerland) . Using the foregoing hydrogel 60, a moisture-containing environment such as that in the interior of a human body may be provided to the vascularized tissue cells 62 and vascular cells 32.

The foregoing hydrogel 60 may be cured by leaving the container 50 for preparation of tissues at a temperature of about 35 to 40°C in an environment of 3 to 10 wt.% carbon dioxide for 20 to 40 minutes.

Then, a cell culture solution 64 is injected to an upper side of the hydrogel. The cell culture solution 64 may be any conventional cell culture fluid, for example, Dulbecco's Modified Eagle's Medium (DMEM) or α-Minimum Essential Medium (α-MEM) may be used.

Accordingly, through a signal material for angiogenesis generated from the vascularized tissue cells 62, new blood vessels 66 may be formed from the vascular cells 32 toward the vascularized tissue cells 62. Further, the vascular cells 32 are bound to the vascularized tissue cells 62 by the new blood vessels 66, thus producing vascularized tissues *in vitro*. In this regard, formation of the new blood vessels 66 that gather around the collection tip 22, can be observed in FIG. 2. As a result, appearance of the new blood vessels 66 is clearly shown, and destruction of the new blood vessels 66 may be easily observed during chemical reagent examination.

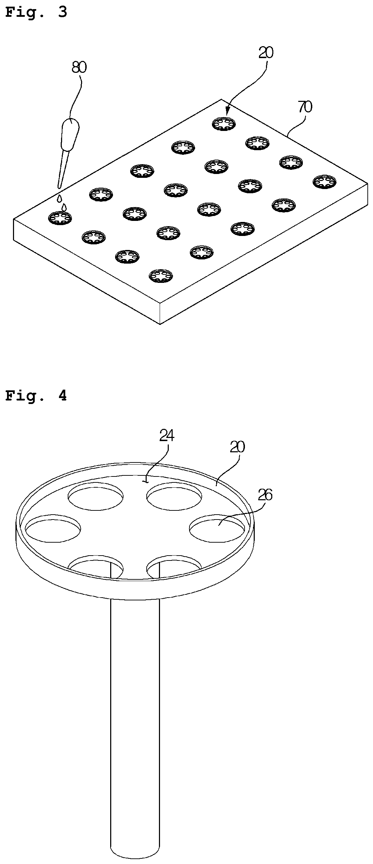

With regard to the obtained vascularized tissues, in order to conduct a variety of examinations such as drug testing or the like, a number of vascularized tissues are simultaneously manufactured on a test panel 70, as shown in FIG. 3. That is, after mounting the container 50 for preparation of tissues on the test panel 70, vascularized tissues are formed in the containers 50 for preparation of tissues, respectively, by the foregoing processes. Accordingly, the vascularized tissues can be manufactured *in vitro* in large quantities.

Further, destruction of the new blood vessels 66 or vascularized tissue cells 62 is observed while varying types or dosages of known chemical reagents introduced into each of the containers 50 for preparation of tissues by a chemical injector 80. Based on the observed results, it is possible to determine an appropriate treatment method or type of chemical reagent for a patient.

Specifically, in order to easily supply a chemical reagent using the chemical injector 80, different methods may be attempted and, for example, a dent 24 may be formed on a top side of the cell collection member 20 and at least one through-hole 26 may be formed in the dent 24, as shown in FIG. 4. According to this, when a chemical reagent is supplied in the dent 24 by the chemical injector 80, the chemical reagent may pass through the through-holes 26 and drop down, thus being directly fed to the *in vitro* vascularized tissues.

As described above, although preferred embodiments of the present invention have been described in the above detailed description, those skilled in the art will appreciate that various modifications and variations are possible without departing from the scope and spirit of the present invention disclosed in the description, and such modifications and variations are dully included within the appended claims.

### [Description of Reference Numerals]

10: container for vascular cells, 12: collection groove
14: inclined part, 20: cell collection member
22: collection tip, 24: dent
26: through-hole, 30, 64: cell culture solution
32: vascular cell, 40: container for mounting
50: container for preparation of tissue, 60: hydrogel
52: vascularized tissue cell, 66: new blood vessel
70: test panel, 80: chemical injector

## Claims

1. A method for manufacturing *in vitro* vascularized tissues, comprising:
supplying a hydrogel mixed with vascularized tissue cells in a container for preparation of tissues;
submerging a collection tip having vascular cells adhered thereto in the hydrogel;
curing the hydrogel while submerging the collection tip therein; and
supplying a cell culture solution to an upper side of the hydrogel.

2. The method according to claim 1, wherein the vascular cells are adhered to the collection tip of a cell collection member by submerging the collection tip in the cell culture solution containing vascular cells in a container for vascular cells.

3. The method according to claim 2, wherein the container for vascular cells has an inclined part to gather the vascular cells around the collection tip.

4. The method according to claim 3, wherein a collection groove for inserting the collection tip therein is formed at the center of the inclined part.

5. The method according to claim 1, wherein the collection tip is made of a porous material or coated with collagen at an outer face thereof.

6. The method according to claim 1, wherein the vascularized tissue cells are at least one selected from cancer cells, brain cells or liver cells.

7. The method according to claim 1, wherein the hydrogel has a cell binding domain while being biodegradable.

8. The method according to claim 1, wherein the hydrogel is a natural hydrogel or synthetic hydrogel.

## Patentansprüche

1. Verfahren zum *in-vitro*-Herstellen von vaskularisierten Geweben, umfassend:
Zuführen eines Hydrogels in einem Gemisch mit vaskularisiertes-Gewebe-Zellen in einen Behälter zur Präparation von Geweben;
Eintauchen einer Sammelspitze mit daran haftenden Gefäßzellen in das Hydrogel;
Härten des Hydrogels, während die Sammelspitze darin eingetaucht ist; und
Zuführen einer Zellkulturlösung an eine Oberseite des Hydrogels.

2. Verfahren gemäß Anspruch 1, wobei die Gefäßzellen an der Sammelspitze eines Zellsammelglieds haften, indem die Sammelspitze in die Gefäßzellen enthaltende Zellkulturlösung in einem Behälter für Gefäßzellen getaucht wird.

3. Verfahren gemäß Anspruch 2, wobei der Behälter für Gefäßzellen einen schrägen Teil aufweist, so dass sich die Gefäßzellen um die Sammelspitze herum sammeln.

4. Verfahren gemäß Anspruch 3, wobei eine Sammelfurche zum Einführen der Sammelspitze darin in der Mitte des schrägen Teils ausgebildet wird.

5. Verfahren gemäß Anspruch 1, wobei die Sammelspitze aus einem porösen Material besteht oder an einer Außenfläche davon mit Kollagen beschichtet ist.

6. Verfahren gemäß Anspruch 1, wobei die vaskularisiertes-Gewebe-Zellen wenigstens eines ausgewählt aus Krebszellen, Hirnzellen oder Leberzellen sind.

7. Verfahren gemäß Anspruch 1, wobei das Hydrogel eine zellbindende Domäne aufweist, wobei es bioabbaubar ist.

8. Verfahren gemäß Anspruch 1, wobei das Hydrogel ein natürliches Hydrogel oder ein synthetisches Hydrogel ist.

## Revendications

1. Procédé de fabrication de tissus vascularisés *in vitro,* comprenant :
la fourniture d'un hydrogel mélangé avec des cellules de tissu vascularisé dans un récipient pour la préparation de tissus
l'immersion d'une pointe de collecte à laquelle des cellules vasculaires sont adhérées dans l'hydrogel
le durcissement de l'hydrogel pendant l'immersion de la pointe de collecte dans celui-ci ; et
la fourniture d'une solution de culture de cellules à un côté supérieur de l'hydrogel.

2. Procédé selon la revendication 1, dans lequel les cellules vasculaires sont adhérées à la pointe de collecte d'un élément de collecte de cellules par immersion de la pointe de collecte dans la solution de culture de cellules contenant des cellules vasculaires dans un récipient pour cellules vasculaires.

3. Procédé selon la revendication 2, dans lequel le récipient pour cellules vasculaires comporte une partie inclinée pour rassembler les cellules vasculaires autour de la pointe de collecte.

4. Procédé selon la revendication 3, dans lequel une rainure de collecte pour insérer la pointe de collecte dans celle-ci est formée au centre de la partie inclinée.

5. Procédé selon la revendication 1, dans lequel la pointe de collecte est constituée d'un matériau poreux ourevêtu de collagène au niveau d'une face externe de celui-ci.

6. Procédé selon la revendication 1, dans lequel les cellules de tissu vascularisé sont au moins l'une choisie parmi des cellules cancéreuses, des cellules cérébrales et des cellules hépatiques.

7. Procédé selon la revendication 1, dans lequel l'hydrogel a un domaine de liaison cellulaire tout en étant biodégradable.

8. Procédé selon la revendication 1, dans lequel l'hydrogel est un hydrogel naturel ou un hydrogel synthétique.
